# EUROPEAN PATENT APPLICATION

(11) **EP 4 019 972 A1**
(43) Date of publication of application: **29.06.2022**
(21) Application number: 21216876.9
(22) Date of filing: 22.12.2021
(51) Int. Cl.: G01N 33/543, G01N 33/569, C12Q 1/18, C12Q 1/00

(54) **METHOD AND SYSTEM FOR THE ELECTROCHEMICAL DETECTION OF MICROBIAL GROWTH OR THE INHIBITION THEREOF**

(30) Priority: 22.12.2020 EP 20216319
(71) Applicant: Universiteit Antwerpen, 2000 Antwerpen (BE)
(72) Inventor: TRASHIN, Stanislav Alexandrovich, 2000 Antwerpen (BE); VANDENHEUVEL, Dieter, 3000 Leuven (BE); LEBEER, Sarah, 2640 Mortsel (BE); DE WAEL, Karolien, 9170 Sint-Pauwels (BE)
(74) Representative: De Clercq & Partners

(57) **Abstract**

The present invention provides a method and electrochemical system for the electrochemical detection of microbial metabolic activity, in particular living microorganism, in a sample or the detection of an antimicrobial compound in a sample using a redox mediator and an electrode system, wherein the metabolic activity or growth of a microorganism (present in or added to the sample) is assessed by measuring an electrical signal, in particular a potential or current output, via the electrode system, which is a measure of the oxidation of a redox mediator (in reduced form) at an electrode. The redox mediator (in oxidized form) is in its turn reduced by the metabolic activity and growth of the microorganism, thus allowing correlating the measured electrical signal with the presence of living microorganisms in the sample or the presence of an antimicrobial compound in the sample.

## Description

### Field of the invention

The present invention relates to an improved method and system for the rapid determination of microbial metabolic activity or living microorganisms in a sample and/or the presence or absence of antimicrobial compounds in a sample.

### Background to the invention

Detection of bacterial growth inhibition is a valuable analytical tool for many industries. In the food industry, bacterial growth inhibition tests are used for the detection of antibiotic residues in food products or food raw materials. Antibiotics are often used in animal farming both as a preventive and therapeutic measure. Strict safety regulations towards the re-entrance of treated livestock and their products in the human food chain were introduced by governments in EU and many countries worldwide. In particular, maximum residue limits (MRLs) for antimicrobial compounds in food products of animal origin have been established by various legislators (such as the EU) to prevent the negative impact of antibiotic residues on human health and on the entire ecosystem. Many sectors are heavily regulated such as meat and see food industries. Cow milk production is also a particularly vulnerable sector in this context and the number of tests for determining antibiotic residues in milk is high. Failure to meet the MRL inadvertently leads to destruction of the production lot. It is thus of vital importance to be able to detect the presence of small amounts of antibiotics accurately and rapidly.

Microbial growth inhibition tests have been developed for the determination of the presence or absence of antibiotic residues in a sample. Examples of such tests include the Delvo^{®} assay (DSM, NL). This test is a ready-to-use test that makes use of a test medium comprising a test organism and a pH indicator. Milk or another liquid is added to the test medium and incubated at 64°C for 3 h 15 min. In the absence of an antibiotic compound, uninhibited growth occurs, which is *inter alia* accompanied by the formation and accumulation of acids in the medium, and the pH indicator changes colour. When an antibiotic compound is present in the sample in a concentration sufficient to inhibit growth of the test organism, little or no microbial growth occurs and the colour of the indicator will stay the same or changes insufficiently, indicating a result above the MRL.

However, the current microbial growth inhibition tests are unable to detect some antibiotic residues at the required MRL - they have a limited sensitivity for certain antibiotics, which is determined by the sensitivity profile of the test organism. This may lead to false negative results, which entails risks for public health, or to false positive results, which results in economic losses. In addition, the current microbial growth inhibition tests also have a long analysis time, i.e. more than 3 h for the Delvo^{®} test. Colour-based bacterial inhibition tests typically rely on acidification of the medium which happens with a delay, because the bacteria need to produce a sufficient amount of acid compounds to shift the pH in the medium and because the sample, such as milk, often intrinsically contains buffering agents as well. Such a delay is undesirable in the processing of fresh products such as milk. Moreover, these tests often do not allow the distinction between basic level infections which can be tolerated and problematic infections by specific pathogenic bacteria.

On the other hand, rapid and sensitive detection of living microorganisms and microbial growth is desirable in many different fields as well. Pathogenic strains of microbes remain an important source of disease in humans. Despite the increasing use of antibiotics infection still often occurs via ingestion of contaminated water or food. Due to the huge impact of foodborne bacterial morbidity on the community, it is important to ensure that consumers receive water and food materials free of potential contamination by pathogens. Microbial contamination may also lead to large production losses. For instance, in Europe many sugar factories store so-called thick juice, a composition with very high sugar content, and, hence an expensive product, for processing after the campaign for periods as long as three to nine months. Taken into account biocide regulatory restrictions, the microbial stability of the thick juice is typically controlled by parameters such as brix (%dry substance), pH and temperature. However, when a serious microbial contamination occurs, there is a significant loss of sugar. These infections are often not noticed until already significant sugar loss has occurred, inter alia due to the contaminating microorganisms not being culturable under standard conditions available in microbiological labs. In the field of fruit juices, many fruit juices, particularly orange juice, are produced cold, and sterilized by filtering, as a heat treatment adversely influences taste and colour. However, some batches may still remain contaminated with yeast, typically below the detection limit of available microbiological techniques.

Bacterial cell concentrations are most often determined by classical plate count techniques. A (pre-treated) sample of the matrix in question (e.g. drinking water, swimming pools, vegetables, meat) is plated on artificial culturing media and incubated for 24-72 h, depending on the bacterial species monitored. This is a lengthy process requiring skilled and trained personnel. Moreover, for many food stuffs with expiration dates in the order of days, the time needed is too long and hence causes both financial (e.g. longer warehousing) and human health risk.

Thus, while testing for pathogens and contaminating microorganisms is standard in many industries, there is still a need for simple methods which provide a quick result with a minimum of reagents.

There is thus a need for improved methods and systems for the rapid, sensitive and accurate detection of microbial activity or small amounts of antimicrobial compounds in a sample.

### Summary of the invention

The present inventors have developed improved methods and systems that address one or more of the above-mentioned problems in the art. The methods and systems of the present invention comprise the use of a redox mediator as an artificial metabolite that, advantageously, can be detected by a cost-efficient electrochemical sensor, allowing continuous *in situ* monitoring.

The improved microbial and antimicrobial detection test described herein relies on the electrochemical detection of the transformation, in particular the transformation rate, of a redox mediator. In the improved methods and systems for the detection of metabolic activity of a microorganism or microbial growth in general and/or the presence of an antimicrobial compound in a sample as envisaged herein, a fluid sample is incubated with an exogenous redox mediator, wherein the redox mediator in oxidized form can be reduced by the metabolic activity and growth of a microorganism, and wherein the redox mediator in reduced form is subsequently oxidized at an electrode, thus generating an electrical signal in an electrode system which signal is proportional to the metabolic activity or microbial growth and the number of the microorganism. Where detection of anti-microbial activity is of interest, the metabolic activity and number of an exogenous microorganism added to the sample will correlate with the presence or absence of an antimicrobial compound in the fluid sample.

Advantageously, the improved electrochemical detection method for metabolic activity of microorganisms, such as for the electrochemical detection of the presence or absence of living microorganisms or antimicrobial compounds in a sample, as described herein is sensitive to the number of microorganisms and their metabolic activity, and does not require changes in the bulk of the growth medium, as is particularly the case for the prior art detection or inhibition tests. Analysis of antimicrobial activity is fast, e.g. 1.5 h or less, and sensitive. The electrochemical detection can be interpreted at the end point of the incubation or in a kinetical, real-time manner, with the latter typically allowing for a more sensitive and more rapid analysis. Advantageously, in particular embodiments, the electrochemical detection method envisaged herein uses a low-cost sensor or electrode system, which is able to monitor in real time the rate of the transformation of the redox mediator.

The improved method can be applied for the detection of living microorganisms in a sample. The improved method can also be applied for the detection of bacterial growth inhibition by antimicrobial compounds, such as antibiotic residues, or by other causes. Advantageously, the improved method allows continuous monitoring of the metabolic activity and growth of the bacteria during incubation, combined with a high sensitivity and low price of the electrochemical sensors.

In a first aspect the invention provides methods of electrochemically detecting metabolic activity of a microorganism or microbial growth, such as in a fluid sample, comprising (a) contacting a fluid sample with an electrode system and a redox mediator, wherein the sample is in contact with the redox mediator and wherein the redox mediator is capable of electron exchange with the electrode system; (b) incubating the sample under conditions suitable for the growth of a microorganism; and (c) measuring an electrical signal, preferably a potential or current output, via the electrode system, wherein the measured electrical signal is a function of the growth or metabolic activity of a microorganism when present in said sample. Because the methods of the present invention are based on the detection of metabolic activity or microbial growth, they can be applied both for the detection of the presence of microorganisms in a sample and for the detection of antimicrobial compounds in a sample. In particular embodiments, the method is used to determine the presence of a microorganism in a sample and the samples are samples suspected of containing a microorganism. In alternative embodiments, the method is used to determine the presence of antimicrobial compounds in a sample and a microorganism is added to the sample that is suspected of containing antimicrobial compounds, so as to determine whether the microorganism can survive in the sample. Stated differently, in general, the present invention thus provides for methods of electrochemically detecting microbial growth or the inhibition thereof, comprising (a) contacting a fluid sample with an electrode system and a redox mediator, and optionally an exogenous microorganism, wherein the sample and the optional exogenous microorganism are in contact with the redox mediator and wherein the redox mediator is capable of electron exchange with the electrode system; (b) incubating the sample under conditions suitable for the growth of a microorganism; and (c) measuring an electrical signal, preferably a potential or current output, via the electrode system, wherein the measured electrical signal is (i) a function of the growth of a microorganism when present in said fluid sample, or (ii) a function of the growth of the exogeneous microorganism or the inhibition thereof when an antimicrobial compound is present in said fluid sample.

Accordingly, where the detection of antimicrobial compounds is envisaged, the fluid sample is further contacted with a microorganism, herein also referred to as an "exogenous microorganism" or "external microorganism", i.e. a microorganism that is not originally present in the fluid sample and that is added to or contacted with the fluid sample prior to the measurement and/or incubation step. In the event an antimicrobial compound is present in the sample the growth or metabolic activity of the added or exogenous microorganism will be affected. In particular embodiments, the method of the invention is thus a method of electrochemically detecting an antimicrobial compound in a fluid sample, and said method further comprises contacting said fluid sample with a microorganism, also referred to herein as an exogenous microorganism, wherein the microorganism is in contact with the redox mediator, incubating the sample under conditions suitable for the growth of said microorganism; measuring an electrical signal, preferably a potential or current output, via the electrode system, wherein the measured electrical signal is a function of the metabolic activity or growth of the microorganism and correlating the measured electrical signal with the presence or absence of an antimicrobial compound in the sample.

Accordingly, in particular embodiments, the invention provides a method of electrochemically detecting an antimicrobial compound in a fluid sample comprising contacting a fluid sample with an electrode system, a redox mediator, and an exogeneous microorganism, wherein the fluid sample and the exogeneous microorganism are in contact with the redox mediator and wherein the redox mediator is capable of electron exchange with the electrode system; incubating the exogeneous microorganism in contact with the sample under conditions suitable for the growth of the microorganism; measuring an electrical signal, preferably a potential or current output, via the electrode system, wherein the measured electrical signal is a function of the metabolic activity or growth of the microorganism; and correlating the measured electrical signal with the presence or absence of an antimicrobial compound in the sample.

Where the detection of microorganisms in a sample is envisaged, the present method of electrochemically detecting living microorganisms in a fluid sample comprises the steps of (a) contacting the fluid sample with an electrode system and a redox mediator, wherein the fluid sample is in contact with the redox mediator and wherein the redox mediator is capable of electron exchange with the electrode system; (b) incubating the sample under conditions suitable for the growth of a microorganism; and (c) measuring an electrical signal via the electrode system, preferably a potential or current output, wherein the measured electrical signal is a function of the growth of a microorganism when present in said sample. Particularly, said method further comprises the step of (d) correlating the measured electrical signal with the presence or absence of a microorganism in the sample.

In particular embodiments of the methods of the invention, the step of measuring an electrical signal (step (b)) comprises applying a voltage to the electrode system and measuring the current output, wherein the measured current output is a function of the metabolic activity or growth of a microorganism when present in the sample or the inhibition of the metabolic activity or growth of an exogeneous microorganism when an antimicrobial compound is present in the sample.

In particular embodiments of the methods of the invention, step (c) comprises the step of measuring the electrical potential between the electrodes, wherein the measured potential is a function of the growth of a microorganism when present in the fluid sample or the inhibition of the metabolic activity or growth of an exogeneous microorganism when an antimicrobial compound is present in the sample.

In particular embodiments, the measuring step (which were applicable can be performed simultaneously with incubation step (b) is performed for a period between 15 min and 3 hr, more particularly for a period between 30 min and 120 min. In certain embodiments, the measuring step, preferably simultaneously with incubation step (b) is performed for a period between 30 min and 90 min.

In particular embodiments, the redox mediator is an oxygen-sensitive redox mediator, preferably a 2-hydroxy-1,4-naphthoquinone or an oxygen-sensitive naphthoquinone derivative, phenazine methosulfate or a phenazine derivative. In alternative embodiments, the redox mediator is an oxygen-insensitive redox mediator, preferably brilliant black, benzoquinone or a benzoquinone derivative, naphthoquinone or 2-methyl-1,4-naphthoquinone, resazurin or a phenoxazine derivative, thionine or a phenothiazine derivatives, isoalloxazine or a flavin derivative, ferrocene or a ferrocene derivative, Osmium bis(2,2'-bipyridine)chloride or an Osmium-containing complex including a polymeric substance.

Where the methods of the invention relate to detecting the presence of a microorganism in a sample, the microorganism can be any microorganism, preferably a bacterium. Where the method of the invention relates to detecting antimicrobial activity in a sample and the method encompasses contacting the fluid sample with a microorganism, such as by adding an exogenous microorganism to the sample, the microorganism is a mesophilic non-spore forming microorganism, such as *Escherichia* sp. or a fermenting lactic acid bacteria, or a thermophilic microorganism, such as a thermophilic *Bacillus* sp. or *Geobacillus* sp. It will be understood that the microorganism added to the sample is one which is susceptible to the antimicrobial compounds suspected to be in the sample.

In particular embodiments of the invention, nutrients are added to the sample to further microbial growth. This will stimulate growth of the microorganism present in or added to the sample. Thus, in particular embodiments, the sample is contacted with one or more nutrients suitable for the growth of said microorganism.

In particular embodiments of the invention the redox mediator is immobilized on the electrode system. Where the method involves contacting a sample to an exogeneous microorganism to determine the presence of antimicrobial activity in the sample, the exogeneous microorganism can be immobilized on the electrode system.

The methods of the invention can be used on a variety of samples such as but not limited to a fluid sample originating from a body liquid, an animal product, an animal tissue, a food product derived from an animal product or an animal tissue, or an aqueous environmental liquid, preferably the sample is blood, urine, wastewater, or milk, more preferably milk.

A further aspect of the invention provides for electrochemical biosensors or devices for detection of metabolic activity or growth of a microorganism in a sample. The electrochemical device comprises an electrode system, a redox mediator capable of electron exchange with the electrode system, and a receptacle configured to contact the fluid sample with the electrode system and the redox mediator. For the detection of antimicrobial compound in a sample, the electrochemical device will further comprise a microorganism, also referred to as an exogenous microorganism, and the device is configured such that the exogeneous microorganism can be in contact with the redox mediator and the sample.

In particular embodiments, the electrochemical device as provided herein is a disposable device, further comprising a support, wherein the electrode system and the receptacle is disposed on the support. In particular embodiments, the receptacle further comprises one or more nutrients for said microorganism. In particular embodiments, receptacle comprises a composition comprising a redox mediator, an exogeneous microorganism and one or more nutrients for the microorganism. The redox mediator may be an oxygen sensitive or oxygen insensitive redox mediator. Advantageously, the redox mediator is an oxygen sensitive redox mediator as further discussed herein, as this increases the sensitivity of the measurements.

A further related aspect of the present invention provides electrochemical detection systems comprising the electrochemical devices of the invention and an electrochemical reader, wherein the electrochemical reader is configured to interact with the electrochemical device as described herein, particularly wherein the electrochemical reader is adapted for the introduction of the electrochemical device within the electrochemical reader.

The electrochemical detection systems as envisaged herein may further comprise one or more of
- a heating element for adjusting the incubation temperature of the electrochemical device as envisaged herein, particularly the heating element is adapted for heating the electrochemical device as envisaged herein, when the electrochemical device is introduced in the electrochemical reader; and/or
- a sampling device for withdrawing a sample from a fluid to be tested and for adding the fluid sample to the receptacle of the electrochemical device provided herein.

The invention further provides methods for electrochemically detecting an antimicrobial compound in a fluid sample as provided herein, wherein the sample is introduced in the electrochemical device as described herein.

### Figure Legends

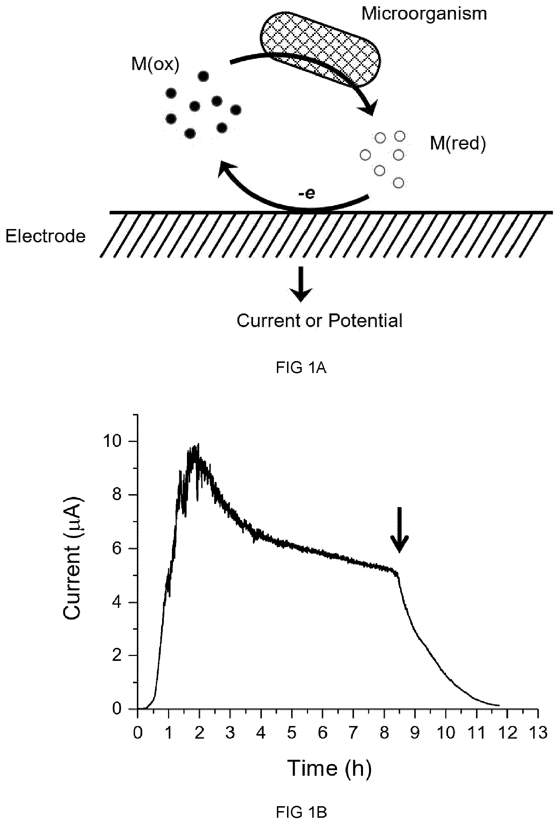

Figure 1 represents the underlying principle of the methods and tools of the present invention. As shown in FIG 1A, the oxidized form of a redox mediator (M(ox)) is reduced due to the growth (metabolic activity) of the microorganism. The reduced form M(red) is oxidized to the oxidized form at the anode, generating a current. Alternatively, the electrode potential can be measured in function of incubation time. Accumulation of M(red) results in shift of the measured potential. FIG 1B represents a measurement in a sample to which a microorganism (*L. rhamnosus,* 3 x 10⁸ CFU/ml) is added. It demonstrates the increase in current over time, when a potential is applied to an electrode system whereby a medium is contacted with the electrode system a redox mediator, particularly in the absence of an antimicrobial compound. The arrow indicates switching off the thermostat (37°C) which puts bacteria in unfavourable conditions (room temperature, approximately 22°C) and, thus, slows their metabolism and growth and results in drop of the current.

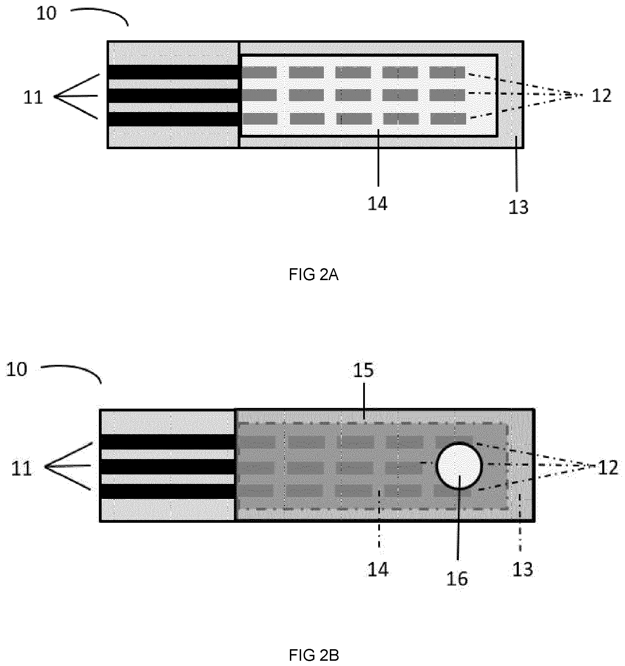

Figure 2 schematically shows an electrochemical device (top view), designed as a disposable sensor, according to an embodiment of the present invention. FIG 2A shows the electrochemical device without a top cover, FIG 2B shows the electrochemical device with a top cover. Dashed lines/structures represent elements which are covered and hidden from a top view by other elements of the electrochemical device.

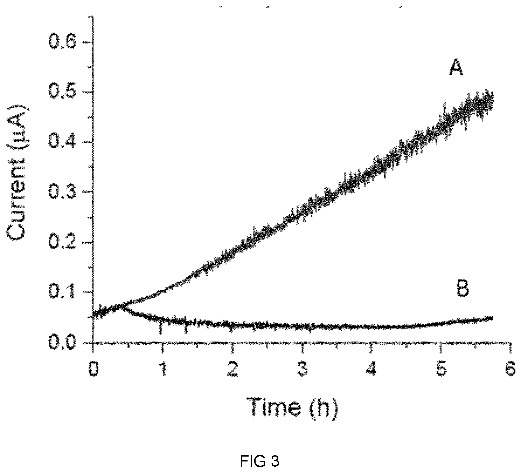

Figure 3 shows the redox mediator induced current in function of incubation time of a microorganism in the absence (A) or presence (B) of an antibiotic compound, for an oxygen insensitive redox mediator according to an embodiment of the invention.

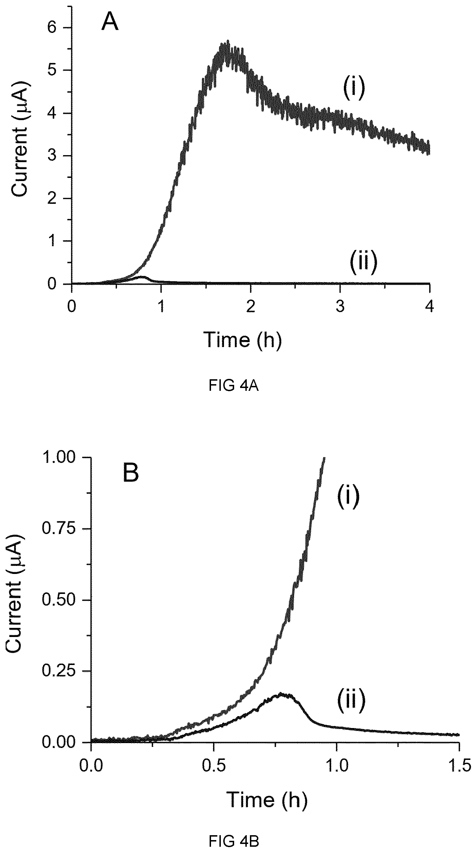

Figure 4 shows the redox mediator induced current in function of incubation time of a microorganism in the absence (i) or presence (ii) of an antibiotic compound, for an oxygen sensitive redox mediator according to an embodiment of the invention. FIG 4A shows changes in currents over 4 h. FIG 4B zooms in on changes over first 1.5 h.

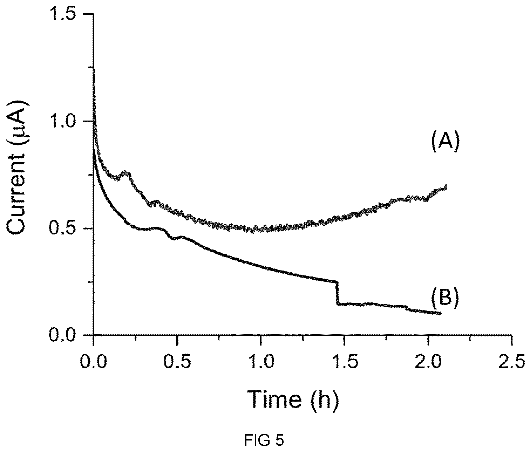

Figure 5 shows the redox mediator induced current in function of incubation time of a microorganism in the absence (A) or presence (B) of an antibiotic compound, for an oxygen sensitive redox mediator using fully disposable screen-printed electrode system inserted in a thermostated housing according to an embodiment of the invention.

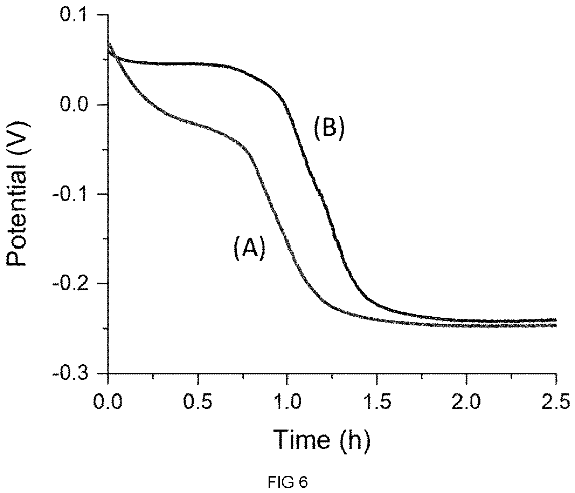

Figure 6 shows the redox mediator induced changes in potential in function of incubation time of a microorganism in the absence (A) or presence (B) of an antibiotic compound, for an oxygen sensitive redox mediator according to an embodiment of the invention.

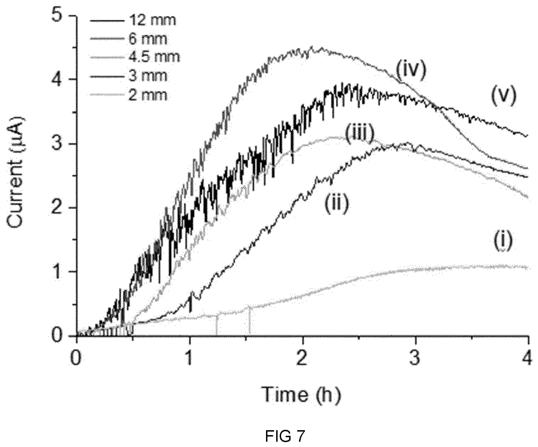

Figure 7 shows the effect of oxygen on the redox mediator induced current in function of incubation time in a miniaturized measuring cell without any mechanical stirring of the solution, by placing five different volumes of liquid medium in the measurement cell. The volumes were 0.15 ml (curve i), 0.25 ml (curve ii), 0.35 ml (curve iii), 0.5 ml (curve iv), 1 ml (curve v) resulting in 2, 3, 4.5, 6 and 12 mm of calculated liquid levels above the working electrode, respectively.

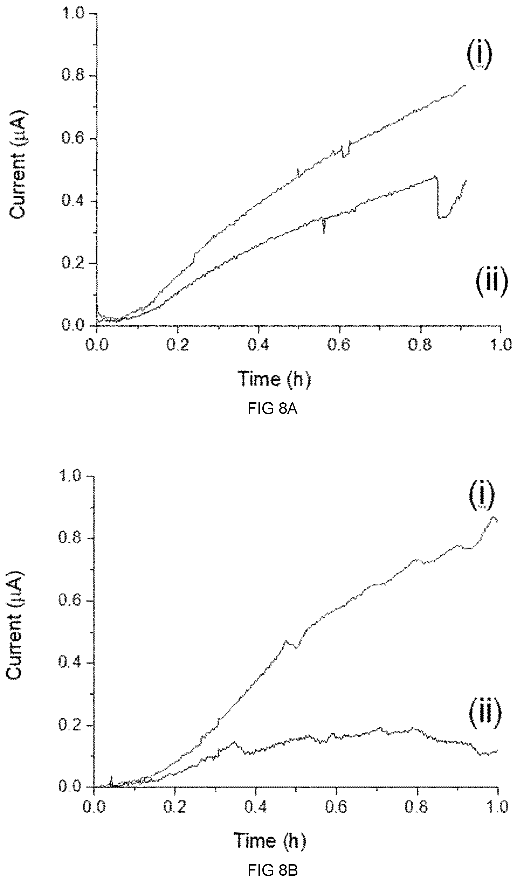

Figure 8 shows the effect of oxygen on the redox mediator induced current in function of incubation time for detection of an antibiotic compound in undiluted raw cow milk. Measurements were conducted either in blank milk (i) or milk spiked with 10 ppm of oxytetracycline (ii), in the presence of an oxygen sensitive redox mediator. The level of the milk in the sensor was 1 ml (A) and 0.35 ml (B), which resulted in no air oxygen interference (A) or significant interference of oxygen (B), in agreement with Figure 7.

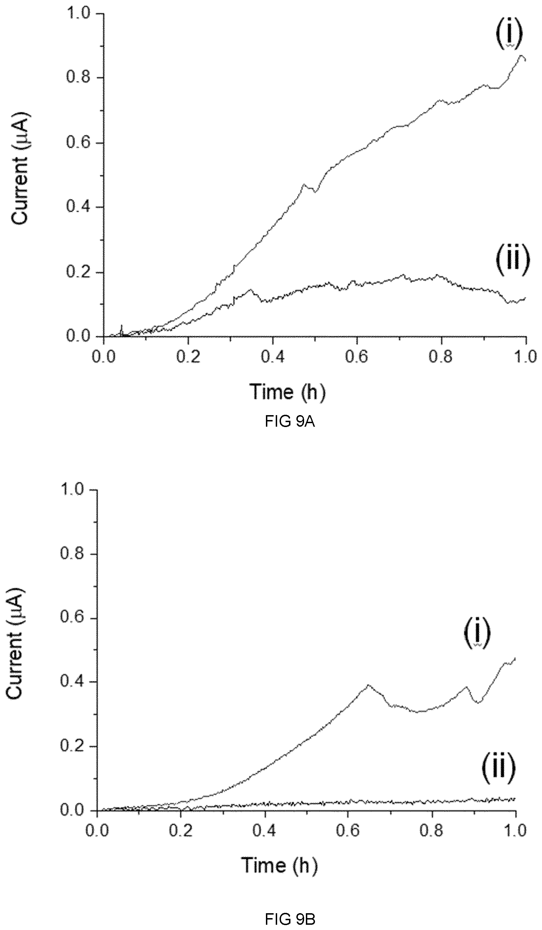

Figure 9 shows the redox mediator induced current in function of incubation time in the detection of an antibiotic compound in undiluted raw cow milk, with different starting number of the cells of the microorganism, i.e. 5·x 10⁸ cells/ml (A) or 2.5 x·10⁸ cells/ml (B) of *Lactobacillus rhamnosus,* in the presence of an oxygen sensitive redox mediator. Measurements were conducted either in blank milk (i) or milk spiked with 10 ppm of oxytetracycline (ii).

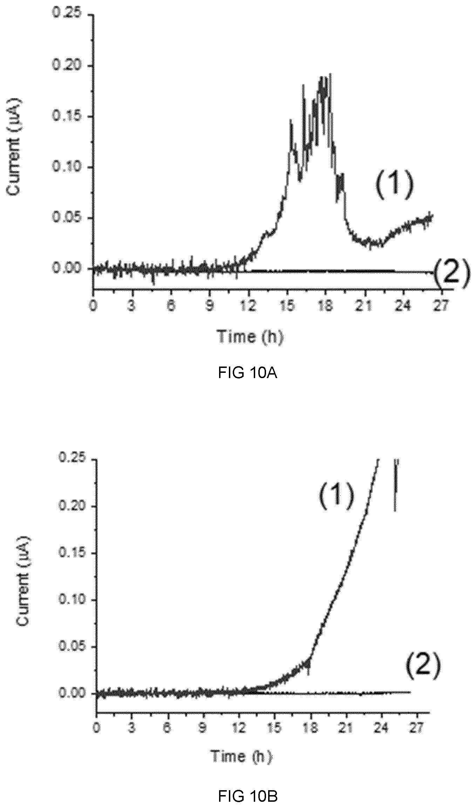

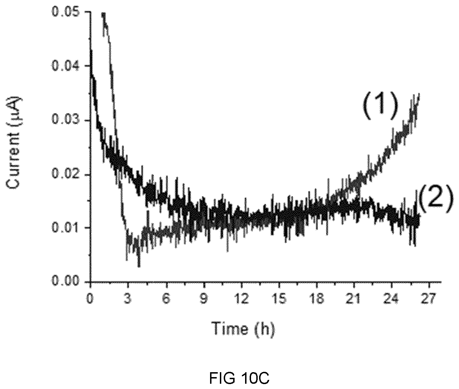

Figure 10 shows the redox mediator induced current in function of incubation time in the detection of bacterial growth in undiluted raw cow milk in the presence of (A) 0.25 mM 2-hydroxy-1,4-naphthoquinone, (B) 0.25 mM 2-methyl-1,4-naphthoquinone or (C) 0.25 mM brilliant black. Bacteria are naturally present in raw cow milk in concentrations about 10⁴ CFU/ml. No additional bacteria were added to the sample. Curve (1) corresponds to raw milk samples and curve (2) corresponds to sterilized controls of the same milk.

### Detailed description of invention

Before the present system and method of the invention are described, it is to be understood that this invention is not limited to particular systems and methods or combinations described, since such systems and methods and combinations may, of course, vary. It is also to be understood that the terminology used herein is not intended to be limiting, since the scope of the present invention will be limited only by the appended claims.

As used herein, the singular forms "a", "an", and "the" include both singular and plural referents unless the context clearly dictates otherwise.

The terms "comprising", "comprises" and "comprised of" as used herein are synonymous with "including", "includes" or "containing", "contains", and are inclusive or open-ended and do not exclude additional, non-recited members, elements, or method steps. It will be appreciated that the terms "comprising", "comprises" and "comprised of" as used herein comprise the terms "consisting of", "consists" and "consists of".

The recitation of numerical ranges by endpoints includes all numbers and fractions subsumed within the respective ranges, as well as the recited endpoints.

The term "about" or "approximately" as used herein when referring to a measurable value such as a parameter, an amount, a temporal duration, and the like, is meant to encompass variations of +/-10% or less, preferably +/-5% or less, more preferably +/-1% or less, and still more preferably +/-0.1% or less of and from the specified value, insofar such variations are appropriate to perform in the disclosed invention. It is to be understood that the value to which the modifier "about" or "approximately" refers is itself also specifically, and preferably, disclosed.

Whereas the terms "one or more" or "at least one", such as one or more or at least one member(s) of a group of members, is clear *per se,* by means of further exemplification, the term encompasses *inter alia* a reference to any one of said members, or to any two or more of said members, such as, e.g., any ≥3, ≥4, ≥5, ≥6 or ≥ 7 etc. of said members, and up to all said members.

All references cited in the present specification are hereby incorporated by reference in their entirety.

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

In the following passages, different aspects of the invention are defined in more detail. Each aspect so defined may be combined with any other aspect or aspects unless clearly indicated to the contrary. In particular, any feature indicated as being preferred or advantageous may be combined with any other feature or features indicated as being preferred or advantageous. Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to a person skilled in the art from this disclosure, in one or more embodiments. Furthermore, while some embodiments described herein include some but not other features included in other embodiments, combinations of features of different embodiments are meant to be within the scope of the invention, and form different embodiments, as would be understood by those in the art. For example, in the appended claims, any of the claimed embodiments can be used in any combination.

In the present description of the invention, reference is made to the accompanying drawings that form a part hereof, and in which are shown by way of illustration only of specific embodiments in which the invention may be practiced. Parenthesized or emboldened reference numerals affixed to respective elements merely exemplify the elements by way of example, with which it is not intended to limit the respective elements. It is to be understood that other embodiments may be utilised and structural or logical changes may be made without departing from the scope of the present invention. The scope of the present invention is defined by the appended claims.

The present invention generally provides methods and systems for detecting metabolic activity of a microorganism and optionally the presence of antimicrobial activity in a sample.

In particular embodiments, the methods and systems of the invention ensure the electrochemical detection of a microorganism in a sample by electrochemically measuring or monitoring the metabolic activity or growth of a microorganism present in said sample. In particular, in these embodiments, a fluid sample is contacted with an exogenous redox mediator in oxidized form, which, when a microorganism is present in the sample, is reduced by the metabolic activity and growth of said microorganism or by reducing compounds produced by the growing microorganism. The redox mediator in reduced form is subsequently oxidized at an electrode generating a signal.

In particular embodiments, the methods and systems of the invention ensures the electrochemical detection or determination of a compound with antimicrobial or microbial metabolic activity inhibiting properties in a sample, by electrochemically measuring or monitoring the metabolic activity of an exogenous microorganism which is contacted with or added to said sample. In particular, in these embodiments, a fluid sample is incubated with an exogeneous microorganism in the presence of an exogenous redox mediator, wherein the redox mediator in oxidized form is reduced by the metabolic activity and growth of the exogeneous microorganism or by reducing compounds produced by the growing exogeneous microorganism. Also, in these embodiments, the redox mediator in reduced form is subsequently oxidized at an electrode generating a signal. The redox mediator thus transfers electrons which arise during the incubation and growth of the microorganism, when present in the sample or when added to the sample, to an electrode, thus generating a signal that is proportional to the metabolic activity of the microorganism. In particular embodiments, where an exogenous microorganism has been added to a sample, the metabolic activity of said microorganism, will in its turn be determined by the presence or absence and the amount of an antimicrobial compound in the fluid sample.

In the context of the present invention, a redox mediator is an organic molecule that is capable of electron exchange with an electrode and which can be reversibly reduced and oxidized. More particularly, the redox mediator can be reduced by the metabolic activity of the microorganism or due to compounds released by the metabolically active and growing microorganism, such as reductive agents or enzymes; and oxidized, particularly at a working electrode. A redox mediator acts as an electron shuttle or an electron transfer agent between the microorganism and the electrode. A redox mediator thus exhibits reversible electrochemistry, in that it can be oxidized and reduced in a reversible manner and in that it is stable in reduced and oxidized forms. This can be tested by means of cyclic voltammetry.

Compounds which form an electrochemically inactive product after reduction or oxidation are thus not considered a redox mediator as envisaged herein. As the redox mediator envisaged herein is not originally present in the fluid sample, the redox mediator as envisaged herein is also referred to as an exogenous redox mediator.

Most generically, the present invention thus provides methods for electrochemically detecting the presence and amount of microbial activity and/or microbial growth in a sample.

The methods of the present invention thus generally comprise (a) contacting the sample with an electrode system and a redox mediator, wherein the sample is in contact with the redox mediator and wherein the redox mediator is capable of electron exchange with the electrode system; (b) incubating the sample under conditions suitable for the growth of a microorganism; and (c) measuring an electrical signal, preferably a potential or current output, via the electrode system, wherein the measured electrical signal is a function of the metabolic activity or growth of a microorganism when present in said sample. As further detailed herein the sample can be a sample suspected to contain a microorganism, whereby the methods allow detection of the presence of microbial growth resulting from the presence of said microorganism. Alternatively, the sample can be a sample suspected to contain an antimicrobial compound, in which case a microorganism susceptible to said antimicrobial compound is added to the sample and the methods allow detection of inhibition of microbial growth of the microorganism resulting from the presence of the antimicrobial compound in said sample.

In particular embodiments, said methods allow the detection of microorganisms in a sample. Such methods are of general interest in the production of food and drinking water. The methods of the present invention provide a reliable, quick detection method of microorganisms within a sample with minimal need for reagents or other infrastructure. In these embodiments, the method of electrochemically detecting living microorganisms in a fluid sample comprises the steps of (a) contacting the fluid sample with an electrode system and a redox mediator, wherein the fluid sample is in contact with the redox mediator and wherein the redox mediator is capable of electron exchange with the electrode system; (b) incubating the sample under conditions suitable for the growth of a microorganism; and (c) measuring an electrical signal via the electrode system, wherein the measured electrical signal is a function of the growth of a microorganism when present in said sample. Particularly, said method further comprises the step of (d) correlating the measured electrical signal with the presence or absence of a microorganism in the sample.

In alternative embodiments of the methods of the present invention the electrochemical detection of microbial growth and activity is used to detect the presence of an antimicrobial compound in the sample. Indeed, upon contacting a sample with an exogenous microorganism, such as upon addition of an exogenous microorganism to a sample, the electrochemical detection of microbial activity will indirectly result in the detection of the presence and amount of an antimicrobial compound in said sample by determining the effect of said antimicrobial activity on a redox mediator using an electrode. In these embodiments, the method is a method of electrochemically detecting an antimicrobial compound in a fluid sample, and said method further comprises (a) contacting said fluid sample with an exogeneous microorganism, wherein the exogeneous microorganism in said fluid sample is in contact with the redox mediator and (b) incubating the sample under conditions suitable for the growth of the exogeneous microorganism; and the method further comprises (d) correlating the measured electrical signal with the presence or absence of an antimicrobial compound in the sample.

An exogenous microorganism as used herein generally refers to a known microorganism which is not originally present in the fluid sample and is added to or contacted with the fluid sample during the incubation step (b) and/or the measurement step (c). It typically is susceptible to the antimicrobial compound suspected in the sample.

The nature of the antibiotic compound is not critical in these methods but will determine the nature of the microorganism added to the sample. Exemplary antibiotic compounds include compounds selected from penicillins, fluoroquinolones, cephalosporins, macrolides, beta-lactams with increased activity (e.g. amoxicillin-clavulanate), tetracyclines, trimethoprim-sulfamethoxazole, lincosamides (e.g. clindamycin), stretogramins, oxazolidinones, lipopeptides, mutilins etc. More specifically, antibiotics selected from amoxycillin, tetracyclin, ceftriaxone, ampicillin, trimethoprim, dihydrostreptamycin, oxytetracycline, kanamycin, penicillin. The skilled person will understand that to test the presence of one or more of these antibiotics in a sample, it is desirable to add a microorganism which is susceptible to the relevant antibiotic. For instance, penicillins have activity against non-beta-lactamase producing gram-positive cocci, including streptococci, enterococci and staphylococci. Amoxycillin acts against a wide variety of gram-positive bacteria but also some gram negative bacteria. Presence of amoxycillin in a sample can be tested, for instance, by adding microorganisms such as E. coli, a streptococcus species or an enterococcus species to the sample. The methods of the invention are of particular interest for the detection of one or more antibiotic compounds which are used e.g. in animal breeding such as tetracyclines and sulfonamides.

It will be understood that the methods for the detection of a microorganism in a sample are of particular interest for those samples where contamination with a microorganism is a risk and needs to be avoided, i.e. samples from processes wherein treatment with antibiotics is not possible or undesired. On the other hand, the methods for the detection of antimicrobial activity of the invention are of particular interest for testing of samples from processes where antibiotics may have been or may have been used, such that they generally do not to contain microorganisms.

More particularly, the methods for electrochemically detecting an antimicrobial compound in a sample comprise
(a) contacting a sample, particularly a fluid sample, with an electrode system, a redox mediator, and an exogeneous microorganism, wherein the exogeneous microorganism is in contact with the redox mediator and wherein the redox mediator is capable of electron exchange with the electrode system;
(b) incubating the microorganism in contact with the sample under conditions suitable for the growth of the microorganism; and
(c) measuring an electrical signal via the electrode system, wherein the measured electrical signal is a function of the growth or metabolic activity of the microorganism; and
(d) correlating the measured electrical signal with the presence or absence of an antimicrobial compound in the sample.

In particular embodiments of the methods envisaged herein, the electrical signal measured in step (c) is a potential or current output. In certain embodiments, step (c) comprises the step of applying a voltage to the electrode system and measuring the current output, wherein the measured current output is a function of metabolic activity or growth of the microorganism when present in the fluid sample or is a function of the metabolic activity or growth of the exogeneous microorganism or the inhibition thereof when an antimicrobial compound is present in said fluid sample. In certain embodiments, step (c) comprises the step of measuring the electrical potential between the electrodes, wherein the measured potential is a function of the metabolic activity or growth of the microorganism when present in the fluid sample or is a function of the metabolic activity or growth of the exogeneous microorganism or the inhibition thereof when an antimicrobial compound is present in said fluid sample.

In particular embodiments, the measurement step is performed sequentially over a given time span. In particular embodiments, particularly where a microorganism is added to the sample, the incubation step (b) and measurement step (c) are performed simultaneously. This way, the electrical signal, particularly a potential or current output, is measured in real time during the incubation during which either inhibited or uninhibited growth of the microorganism takes place.

As illustrated in FIG 1A, in the methods and systems of the present invention, the measured electrical signal originates from the oxidation of the reduced form of the redox mediator at an electrode. The oxidized form in its turn is reduced due to the growth or metabolic activity of the microorganism, when present and not inhibited in the sample. The measured electrical signal thus correlates with the turn-over of the different oxidation states of the redox mediator, which in its turn is correlated with the metabolic activity and growth of the microorganism. FIG 1B demonstrates an exemplary measurement of an electrical signal in a medium when a microorganism is added thereto. The arrow indicates the moment when conditions change from favourable (37°C) to unfavourable (22°C) for bacterial growth and metabolism, which results in immediate drop of the signal measured. This demonstrates the ability to detect living microorganism and microbial metabolism in a sample as well as the ability to distinguish between uninhibited metabolic activity and growth in the absence of an antimicrobial compound in the sample, and arrested metabolism and growth in the presence of an antimicrobial compound.

Colorimetric methods for the determination of microorganisms or the presence of an antimicrobial compound in a sample, which involve a visual or photometric evaluation, typically at the end of a given incubation time of the sample, rely on a change of the properties, particularly the pH, of the bulk of the test medium, and thus require that a sufficient amount of metabolites, particularly acids, is accumulated in the medium. In contrast, the electrochemical determination envisaged herein has the advantage that the electrochemical reaction yields an electric signal (e.g. a current output), which is directly related to the growth and metabolic activity of the living microorganism (via the electron transfer by the redox mediator), and is thus not dependent on the accumulation of metabolites. Advantageously, the electrochemical determination envisaged herein can be measured in real-time during an incubation period. This further reduces the likelihood of false positive or false negative results.

Accordingly, in certain embodiments measurements are performed over time, such as for a period between 15 min and 3 h, or for a period between 30 min to 120 min, such as for a period between 30 min and 90 min, or between 30 min and 60 min. This is of particular interest in the detection of antimicrobial compounds, where a microorganism is added to the sample. In these embodiments, measurements can take place during incubation. Thus, in particular embodiments of the methods for the detection of antimicrobial compounds described above, incubation step (b) and measurement step (c) are performed simultaneously for the duration of step (b), in particular for a period between 15 min and 3 h, or for a period between 30 min to 120 min, such as for a period between 30 min and 90 min, or between 30 min and 60 min.

Likewise, performing measurement step (c) simultaneously with incubation step (b) is also of interest for the detection of living microorganisms in a sample. Thus, in particular embodiments of the methods for the detection of living microorganism in a sample described above, incubation step (b) and measurement step (c) are performed simultaneously for the duration of step (b), in particular for a period between 15 min and 3 h, or for a period between 30 min to 120 min, such as for a period between 30 min and 90 min, or between 30 min and 60 min. The electrode system as envisaged in the different aspects and embodiments of the present invention comprises one or more working electrodes and a counter electrode and/or a reference electrode. In certain embodiments, the electrode system comprises one or more working electrodes and a counter electrode. In certain embodiments, the electrode system comprises one or more working electrodes and a reference electrode. In certain embodiments, the electrode system comprises one or more working electrodes, a counter electrode and a reference electrode. In certain embodiments, the reference electrode and the counter electrode may be the same electrode.

The redox reaction that is detected in the presence of a microorganism, i.e. the oxidation of the reduced form of the redox mediator to its oxidized form, occurs at the surface of the working electrode, and comprises the transfer of electrons from the oxidized form of the redox mediator to the working electrode, particularly when a potential is applied to the electrode system, particularly to the working electrode compared to the reference electrode and/or to the counter electrode. When a reference electrode is present, it typically has a constant and well-known potential, such as e.g. an Ag/AgCI electrode. In particular embodiments, a pseudo-reference (also called quasi-reference) electrode is used. These reference electrodes provide reproducible and stable potential in given conditions and used in the same way as usual reference electrodes. The potential of a quasi-reference electrode is known approximately and will vary dependent on the solution (for example can vary depending on the amount of chloride in solution).

Well-known electrically conductive materials can be used for the electrodes. In particular embodiments, the electrode system comprises carbon- or silver-based electrodes. Advantageously, carbon electrodes are cheap and easy to manufacture, and are particularly suitable for use in disposable sensors according to the present invention, as further discussed herein. In addition, carbon and silver electrodes are typically more selective to the redox mediator, and are less influenced by other compounds or metabolites present in the test composition. The electrodes may also contain other noble metals such as gold. Platinum based electrodes are generally less preferred, as they are expensive and their reaction with other components may interfere with the oxidation of the redox mediator. Thus, different factors will affect the selection of a suitable electrode system, including the selection of the redox mediator. In particular embodiments, the electrodes are screen-printed electrodes, whereby the electrodes and contacts are made of thin layers of carbon or silver-based inks.

In particular embodiments, the methods of the present invention envisage contacting a sample (which may comprise a microorganism) with an electrode system and a redox mediator. Where the focus of the methods of the invention is on the detection of antimicrobial compounds in a sample, an external or exogenous microorganism is added, which is known to be susceptible to the (one or more) antimicrobial compounds of interest. In these embodiments the methods of the present invention envisage contacting a sample with an electrode system, a redox mediator, and an external or exogeneous microorganism, in particular with a known and specified amount of said external or exogeneous microorganism, as further described herein below.

Suitable systems wherein a sample can be contacted with an electrode system (as well as a redox mediator and, where applicable, a microorganism) can be envisaged by the skilled person and are described herein below.

In certain embodiments, the redox mediator and/or the microorganism may be immobilized on the electrode system.

As envisaged herein, the redox mediator is a reversible oxidisable and reduceable organic and exogeneous compound, which is capable of being electron transfer with an electrode. In particular, the redox mediator is converted in its reduced form due to the metabolic activity of a/the microorganism present in or contacted with, such as added to the sample or due to compounds released by the microorganism and wherein its reduced form is capable of being converted in its oxidized form by the electrode system in step (c).

Many redox mediators or electron carriers are known in the art. Suitable redox mediators for the methods and systems envisaged herein are redox mediators that are not toxic to the microorganism used in the methods and systems envisaged herein. Furthermore, a suitable redox mediator is so chosen and selected that it is converted rapidly and selectively by the electrode system used. Suitable redox mediators, particularly suitable redox mediator and electrode systems, may be selected by cyclic voltammetry, as known in the art, which provides information on the electrochemical reaction rates of the oxidized and reduced form of the redox mediator and the useful potential range for electrochemical oxidation of the reduced form of the redox mediator for a certain electrode system.

In certain embodiments, the redox mediator is a quinone or quinone derivative, in particular an imine or benzoquinone or benzoquinone derivative, naphthoquinone or naphthoquinone derivative, or anthraquinone or anthraquinone derivative. Preferred quinone derivatives include hydroxyquinones, such as hydroxybenzoquinone, hydroxynaphthoquinone or hydroxyantraquinone, wherein any number (n) of hydrogen atoms have been replace by (n) hydroxyl functional groups, with n = 1, 2, 3, 4 or more. In certain embodiments, the redox mediator is brilliant black. In further embodiments, the redox mediator is a phenazine, phenoxazine or phenotiazine such as phenazine methosulfate, resazurin or thionine respectively, or a phenazine, phenoxazine or phenotiazine derivative. In further embodiments, the redox mediator is a flavin (such as isoalloxazine). In further embodiments the redox mediator is a ferrocene or a ferrocene derivative. In particular embodiments the redox mediator is an Osmium-containing complex (e.g., Osmium bis(2,2'-bipyridine)chloride) or an Osmium containing polymer. Suitable redox mediator compounds are known in the art and have been described, i.a. by Fultz and Durst 1982 (Anal. Chim. Acta 140(1):1-18).

In certain embodiments, the sample, microorganism and electrode system are contacted with the redox mediator in a concentration ranging from 0.01 - 10 mM, particularly in a concentration range of 0.5 - 5 mM, more in particular in a concentration range of 0.1 - 1 mM.

In certain embodiments, the redox mediator is not sensitive to oxygen or the method is performed under oxygen-limiting conditions. Examples of an oxygen-insensitive redox mediator include brilliant black, 2-methyl-1,4-naphthoquinone, naphthoquinone, benzoquinone and their derivatives. Oxygen-insensitive redox mediators may be particularly useful in methods for the detection of the growth of facultative anaerobic microorganisms or aerotolerant anaerobic microorganisms which are able to grow in the absence of oxygen, and are thus less affected by oxygen depletion in the medium. This contributes to the robustness of the methods envisaged herein.

In certain embodiments of the methods envisaged herein, particularly in the methods for the detection of antimicrobial activity, the redox mediator may be sensitive to oxygen, more specifically, the reduced form of the mediator can be oxidized by oxygen. Typically, in this case, the method is performed in contact with air. Advantageously, the use of an oxygen sensitive mediator may increase the sensitivity of the methods envisaged herein towards detection of the inhibition event and/or speed of the methods envisaged herein, in particular, due to possible increase in starting amount of the microorganism. A particularly preferred embodiment thus relates to a method of electrochemically detecting an antimicrobial compound in a fluid sample, wherein the redox mediator is an oxygen-sensitive redox mediator.

In the presence of an oxygen sensitive redox mediator, re-oxidation of the reduced form of the redox mediator by oxygen can significantly increase the sensitivity of the method towards detection of antimicrobial substances. During incubation, oxygen is consumed by the microorganism's aerobic metabolism, which results in the biochemical reduction of the mediator. The re-oxidation of the reduced form of the oxygen sensitive mediator also consumes oxygen. When the oxygen level drops, the mediator appears in significant amount in the reduced form and gives rise to the electrochemical response as it is re-oxidized at the anode.. When the microorganism is insufficient in cell number due to the presence of antimicrobial substances or its metabolism was slowed by antimicrobial substances, the oxygen level in solution stays high due to flux of oxygen from air. This prevents formation of the reduced form of the mediator and appearance of the electrochemical response in full or in part. Examples of an oxygen sensitive redox mediator include 2-hydroxy-1,4-naphthoquinone and phenazine methosulfate. Naphthoquinone derivatives vary in re-oxidation kinetics by oxygen (Free Radical Research, 32 (2000), 245-253). For example, unsubstituted naphthoquinone or 2-methyl-1,4-naphthoquinone exhibits much slower re-oxidation kinetics compared to 2-hydroxy-1,4-naphthoquinone and can be considered as examples of oxygen insensitive mediators. In the case of a faster re-oxidation kinetics, an oxygen level has to decrease to lower values to allow the appearance of the reduced mediator in the solution compared to the case of slower re-oxidation kinetics. If a short detection time is desirable, the structure or the choice of the oxygen sensitive mediator can be optimized for the given embodiments, e.g., design of the electrochemical test system, permeability of air oxygen into the sample solution, metabolism rate and starting amount of the exogenous microorganism, and its required sensitivity to certain antimicrobial compounds.

Compared to the embodiments when using an oxygen insensitive mediator and/or when access of oxygen from air is restricted, the use of an oxygen sensitive mediator in combination with access of oxygen from air enlarges the difference in electrical signal, such as the electrical current output, between the control sample not containing an antimicrobial compound and a sample containing an antimicrobial compound, thus increasing the sensitivity of the method and allowing shorter incubation times as described in Example 6 and shown in FIG 8. Inhibition of the metabolism and/or decrease in the number of microorganisms will reduce the rate of generation of the reduced form of the mediator and additionally increase the amount of available oxygen that will oxidize all or a part of the reduced form of the mediator before the mediator reaches the electrode surface resulting in a drop of the measured signal (returning of the signal towards the values at the beginning of measurements). When starting amount of test microorganisms is sufficiently high, the use of the oxygen sensitive mediator allows to see high activity of the microorganism in the beginning of the measurements followed by an inhibition if an antibiotic is present in the sample as shown in FIG 4B and 8B. Importantly, this scheme excludes the need in a separate control sample (without antibiotic), and can ensure that the sensor was stored and operated correctly or, in other words, can exclude false positive measurements due to a theoretically possible sensor malfunction, thus, making the methods envisaged herein to be more reliable.

The effect of oxygen can be regulated by either using controllable stirring of the measuring solution exposed to air and/or by adjusting the level of liquid medium separating the working electrode and air. By adjusting the level of liquid, the effect of oxygen can be regulated without additional mechanical stirring in solution as shown in FIG 7. The oxygen flux can be also regulated by a suitable design of the measuring cell, for example, by using caps with or without holes, membranes with different O₂ permeability and dead air volume under a cap of a measuring cell.

The methods for the detection of a microorganism according to the present invention can be used for the detection of any micro-organism of interest. More particularly the detection of the presence of pathogenic bacteria is envisaged such as *Escherichia coli* (*E. coli*)*, Staphylococcus aureus* (*S. aureus*) and *Salmonella typhimurium* (*S. typhimurium*)*.* The methods of the invention will detect the growth of any bacterium in a sample which can ensure reduction of a redox mediator by its growth or metabolic activity.

Also, for the methods and systems of the invention which relate to detecting antimicrobial activity and involve adding a microorganism to a sample, multiple microorganism species can be used. In these embodiments, it will be necessary to determine that these microorganisms are susceptible to the antimicrobial compounds of which detection is envisaged. Typically, the antibiotics of interest are broad-spectrum antibiotics such as penicillin, which is used in the treatment and prevention of mastitis in cows. Other antibiotics that have been detected in milk include but are not limited to sulfonamids, tetracyclins, streptomycin, lyncomicin, erythromycin, tylosin, gentamycin and neomycin.

In certain embodiments of the methods for the electrochemical detection of antimicrobial compounds, the external or exogenous microorganism used in the methods for detecting antimicrobial compounds is a mesophilic non-spore forming microorganism, particularly the microorganism is an *Escherichia* sp. or a fermenting lactic acid bacteria. *Escherichia* sp., such as *E. coli* are able to use electron transport chains or oxidoreductases in both respiration and fermentation, thus ensuring a rapid interaction with the redox mediator. Facultative anaerobic microorganisms, such as *Escherichia* sp., or aerotolerant anaerobic microorganisms, such as the fermenting lactic acid bacteria, are able to grow in the absence of oxygen, and are thus less affected by oxygen depletion in the medium, thus contributing to the robustness of the methods envisaged herein.

Non-limiting examples of bacteria envisaged for use in the methods provided herein, particularly as external or exogenous microorganism for use in the methods for electrochemically detecting an antimicrobial compound as envisaged herein, are *Geobacillus stearothermophilus, Lactobacillus rhamnosus, Bacillus cereus, Bacillus subtilis, Bacillus pumilus, Escherichia coli, Kocuria rhizophila, Yersinia ruckeri,* and *Streptococcus salivarius* ssp. thermophiles. In certain embodiments, the microorganism may be a spore-forming microorganism, such as a spore-forming *Bacillus* sp. In certain embodiments, the microorganism is a thermophilic microorganism, such as a thermophilic *Bacillus* sp. or *Geobacillus* sp., or a thermophilic *Streptococcus* sp, such as *Geobacillus stearothermophilus.*

In certain embodiments, the number of microorganism detected in or added to the sample is expressed as Colony Forming Units per ml of said test composition (CFU/ml) i.e. the number of microorganisms, vegetative cells and/or spores thereof, capable of producing colonies of microorganism.

In particular embodiments of the methods and systems for detecting an antimicrobial compound in a sample as described herein, the number of microorganisms used, i.e. the number of microorganisms contacted at the outset with the sample ranges between 10⁵ and 10¹² CFU/ml, preferably between 10⁶ and 10¹⁰ CFU/ml or between 2 x 10⁶ and 10⁹ CFU/ml, more preferably between 5 x 10⁶ and 5 x 10⁸ CFU/ml, or between 10⁷ and 10⁸ CFU/ml.

As detailed above, the methods of the present invention envisage (a) contacting a fluid sample with an electrode system and a redox mediator, wherein the sample is in contact with the redox mediator and wherein the redox mediator is capable of electron exchange with the electrode system; (b) incubating the sample under conditions suitable for the growth of a microorganism and (c) measuring an electrical signal, preferably a potential or current output, via the electrode system, wherein the measured electrical signal is a function of the metabolic activity or microbial growth of a microorganism when present in said sample. The microbial growth can be taken as an indication of the presence of a microorganism in a sample, or as an indication of the inhibition of microbial growth of an external microorganism added to the sample due to the presence of an antimicrobial compound said sample.

Accordingly, the methods of the present invention involve contacting a sample with an electrode system and a test composition. Where the method envisages detecting living microorganisms or bacterial metabolism in a sample, the test composition comprises a redox mediator. Where the method envisages detecting an antimicrobial compound, the test composition comprises a redox mediator and/or an exogeneous microorganism. It is understood that the latter can be added from the start or during the measurements. In particular embodiments, the test composition comprises both the redox mediator and the microorganism as envisaged herein. The test composition furthers the contacting of the sample with the microorganism and of the redox mediator with the microorganism and the electrode system. It is understood that, for carrying out the detection methods provided herein, the test composition not only comprises the redox mediator and/or the micro-organism but is in contact with the electrode system, so that the redox mediator can interact with the microorganism and is capable of transferring electrons to the electrode system.

The test composition as envisaged herein may be in the form of a liquid (solution), a solid or in the form of a sol or a gel. In certain embodiments, the redox mediator is present in the test composition in a concentration ranging from 0.01 -10 mM, particularly in a concentration range of 0.5-5 mM, more in particular in a concentration range of 0.1 - 1 mM.

It will be understood to the skilled person that the methods of the present invention are preferably carried on a medium that is sufficiently liquid to ensure exchange of electrons between the sample, microorganism (added or present therein), redox indicator and electrode system. This implies that the combination of the test composition and the sample results in a fluid medium. In particular embodiments, the test composition can be a fluid to which either a fluid sample or a dry sample is added. In particular embodiments, the components of the test composition are provided as dried/dry ingredients and are dissolved in the liquid of the fluid sample as detailed below. The inverse situation can also be envisaged.

Where the methods involve contacting the sample with an exogenous microorganism, the amount of microorganism added may vary. In certain embodiments, the microorganism in the test composition ranges between 10⁵ and 10¹² CFU/ml, preferably between 10⁶ and 10¹⁰ CFU/ml or between 2 x 10⁶ and 10⁹ CFU/ml, more preferably between 5 x 10⁶ and 5 x 10⁸ CFU/ml, or between 10⁷ and 10⁸ CFU/ml.

In the methods of the present invention, bacterial growth, either growth of the bacteria to be detected in the sample or growth of the bacteria added to the sample for detection of an antimicrobial compound, can be stimulated by addition of nutrients or other compounds which have this effect. Thus, in particular embodiments, the test composition further comprises one or more nutrients for the microorganism. The term "nutrient" refers to one or more nutritive substances or ingredients that promote and/or are required for the growth of microorganisms as used in the different embodiments of the present invention. In certain embodiments, a test composition comprising one or more nutrients, particularly in liquid or gel form, thus forms an incubation medium for the microorganism, allowing for the growth of said microorganism. Optionally, the test composition further comprises stabilizers, salts, pH adjusting agents and/or buffering agents, and/or viscosity-increasing agents. pH adjusting agents and buffering agents set the pH value of the test composition to the required value, suitable for growth of the microorganism. Suitable viscosity-increasing agents are known to the skilled person and include carboxymethyl cellulose and other cellulose derivatives, maltodextrins, polyacrylamide, polyvinyl alcohol, xanthan gum or other gums, and the like

In certain embodiments, the test composition is in the form of a gel, and may comprise a gelling agent. Suitable gelling agents are known to the skilled person and include agar, alginic acid and salts thereof, carrageenan, gelatin, hydroxypropylguar and derivatives thereof, locust bean gum (Carob gum), and the like.

In certain embodiments, the test composition is in the form of a solid matrix, such as obtained by drying a liquid test composition, and may comprise a suitable carrier material, such as cotton, metal particles, paper, polymers or biopolymer, silicates, sponges and the like.

In certain embodiments, upon application of the liquid sample, the test composition in solid form rehydrates and is dissolved in the liquid sample.

In certain embodiments, the redox mediator and/or the microorganism, where applicable, such as in the form of a test composition as envisaged herein is/are immobilized on an electrode of the electrode system.

In the methods for detection of an antimicrobial compound provided herein, where the microorganism and the redox mediator are in contact with each other, such as in a test composition, prior to the contacting with the sample it is particularly preferred that there is no or minimal growth of the microorganism prior to the addition of the fluid sample. This may be achieved in multiple ways, particularly by storing and keeping the microorganism or the test system or parts thereof, such as the electrochemical device and/or test composition comprising the microorganism as envisaged herein, under conditions unsuitable or unfavourable for the growth of the microorganism, such as storage at an unfavorable temperature and/or in the absence of nutrients essential for the growth and metabolic activity of the test organism. In certain embodiments, the test composition comprising the microorganism and optionally the redox mediator may be provided as a dried composition, whereby the addition of the fluid sample results in the hydration and dissolution of the test composition, allowing the growth of the microorganism.

Particular embodiments of the methods of the invention, as detailed herein, allow to determine the presence of an antimicrobial compound in a fluid sample. The antimicrobial compound to be detected can be any antimicrobial or antibiotic compound, including but not limited to a beta-lactam antibiotic compound, a tetracycline antibiotic compound, a sulfonamide antibiotic compound, an aminoglycoside antibiotic compound, a macrolide antibiotic compound, a quinoline antibiotic compound, and the like. As detailed above, the nature and specificity of the antibiotic compound will determine the nature of the microorganism used in these methods.

As detailed above, the sample to be analysed by the methods of the present invention will differ depending on whether detection of microbial activity or detection of antimicrobial compounds is envisaged.

The sample to be analysed by the methods for the detection of a living microorganism as envisaged herein may originate from any source where microorganisms may be present or suspected to be present (but are typically undesired), such as from water sources and water treatment facilities as well as wastewater. In certain embodiments, the sample originates from food or beverage products, vegetable or animal based food ingredients, animal products or tissues, body liquids and the like. Sampling and preparation of the samples are known to the skilled person. Certain embodiments of the methods as envisaged herein thus provide for a method for the electrochemical detection of a living microorganism in a food or beverage product, in a vegetable or animal based food ingredient, in an animal product, tissue or a body liquid, or in a water source, wherein the method further comprises, prior to steps (a)-(c) the step of preparing a fluid sample from said food or beverage product, from said vegetable or animal based food ingredient, from an animal product, tissue or body liquid, or from a water source or water treatment facility.

The sample to be analysed by the methods for the detection of an antimicrobial compound may originate from any source where antibiotic residues or other antimicrobial compounds may be present or suspected to be present, in particular from animal products or animal tissues, food products comprising such animal products or tissues, body liquids or aqueous environmental liquids, such as wastewater.

Certain embodiments of the methods as envisaged herein thus provide for a method for the electrochemical detection of an antimicrobial compound in a food or beverage product, in a vegetable or animal based food ingredient, in an animal product or tissue, in a body liquid or in an aqueous environmental liquid, wherein the method further comprises, prior to steps (a)-(c), the step of preparing a fluid sample from said food or beverage product, from said vegetable or animal based food ingredient, from an animal product, tissue or body liquid or from an environmental aqueous liquid.

In certain embodiments, the fluid sample may be derived from animal products or animal tissues, or in food products comprising such animal products or tissues. Examples include milk and processed milk products, meat, organs and processed meat products, eggs, fish and sea food products, such as shrimps; and the like.

In certain embodiments, the fluid sample originate from a body liquid or an animal tissues, for instance a body liquid or animal tissue which is suitable for examination by the authorities. Examples are blood, kidney tissue and urine.

In certain embodiments, the fluid sample originates from an aqueous environmental liquid, such as wastewater or process water used or disposed of in any type of industry.

In a preferred embodiment, the sample is urine, blood, egg, meat, liver, fish, shrimp, feed and/or milk.

In a particularly preferred embodiment, the sample is milk. The milk can be derived from cows, but also from sheep, goats, or other animals.

In certain embodiment, when the product to be tested is not a fluid, a fluid sample potentially comprising the microorganism or the antimicrobial compound can be obtained by extraction of the product.

In the context of the methods for detecting an antimicrobial compound of the present invention, the sample is in practice typically contacted with the microorganism, redox mediator and electrode system as described herein by providing the sample in a receiving means, such as a receptacle or a cavity within a system which is suitable for this purpose. The sample may be provided in this receiving means with the aid of a sampling device, which may be a container, optionally with volume markings, such as a syringe or pipette.

In the case of a method for electrochemically detecting an antimicrobial compound in a sample as envisaged herein, after contacting the sample with the exogenous micro-organism and the redox mediator, in step (b), the microorganism in contact with the sample is incubated under conditions suitable for the growth of the microorganism and for a period sufficiently long for the microorganism to grow in case no antibiotic is present. This period can be determined by including a control sample in the assay, i.e. a sample that is antibiotic free. Additionally or alternatively, the growth of the microorganism, inhibited or not, may be continuously monitored by measuring the electrical signal.

In the case of a method for electrochemically detecting a microorganism in a sample as envisaged herein, after contacting the sample with the redox mediator, in step (b), the sample is incubated under conditions suitable for the growth of a microorganism and for a period sufficiently long for the microorganism to grow. This period can be determined by including suitable control sample in the assay, i.e. one or more samples that comprise a relevant amount of a relevant microorganism. Additionally or alternatively, the growth of the microorganism may be continuously monitored by measuring the electrical signal.

Growth of the microorganism, either exogenous or present in the sample, may be induced by adding nutrients, optionally before the contacting of said sample, and/or raising the temperature, and/or providing for a pH-value at which the exogenous microorganism is able to grow.

In certain embodiments, the incubation of the microorganism in contact with or present in the sample and with the redox mediator occurs for a period between 15 min and 3 hr, or for a period between 30 min to 120 min, such as for a period between 30 min and 90 min, or between 30 min and 60 min, particularly at a temperature of about the optimal growth temperature of the microorganism. A mesophilic microorganism is preferably incubated between 25 °C and 45° C, such as between 30 °C and 40 °C. A thermophilic microorganism is preferably incubated between 50 °C and 70 °C, such as between 60 °C and 65 °C.

In the methods envisaged herein, the amount of growth and metabolic activity of the microorganism is monitored by measuring an electrical signal, preferably a potential or current output, via the electrode system. In particular embodiments, a current output is measured when a potential is applied to the electrode system. Due to the metabolic activity and/or the production of reducing substances, the redox mediator is converted in its reduced form, which in its turn is reoxidized to the initial compound at an electrode, by transferring electrons to the electrode, thus generating a current in the electrode system.

In the methods for the detection of a microorganism in a sample, the measured electrical signal, particularly the measured current output, is correlated with the presence or absence of a microorganism in the sample. This may be performed by assessing the value of the measured signal, particularly the measured current output. In particular embodiments, the measured current output may be detected over time. In particular embodiments, the methods may involve adding an antimicrobial compound and using kinetic methods for evaluating the impact of the antimicrobial compound on the electrical signal. In these embodiments, the output can be measured before and after addition of the antimicrobial compound or continuously.

In the methods for the detection of an antimicrobial compound, the measured electrical signal, particularly the measured current output, is correlated with the presence or absence of an antimicrobial compound in the sample. This may be performed by assessing the value of the measured signal, particularly the measured current output, after a certain incubation time of the sample with the microorganism and the redox mediator, and/or by using kinetic methods for evaluating the presence/absence of an antimicrobial compound based on the electrical signal, particularly the measured current output, continuously measured during at least part of the incubation period of the sample with the microorganism and the redox mediator, particularly continuously measured during the full length of the incubation period. As discussed above, in these embodiments, an oxygen sensitive redox mediator may be particularly used.

Another aspect of the present invention relates to an electrochemical biosensor or device for detecting a microorganism or detecting an antimicrobial compound in a sample. The device of the invention however differs from traditional biosensors in that they directly detect microbial growth.

In a particular embodiment, the electrochemical device as envisaged herein is developed for the detection of an antimicrobial compound in a sample. Such an electrochemical device according to the invention comprises an electrode system and means for contacting a sample with an exogenous microorganism and a redox mediator which itself can be in contact with the electrode system. In particular embodiments, the electrochemical device further comprises an exogenous microorganism and a redox mediator. In particular embodiments, the electrochemical device comprises an electrode system; a redox mediator, a microorganism, and a receptacle configured to contact the fluid sample with the electrode system, the redox mediator and the microorganism, wherein the microorganism is in contact with the redox mediator and wherein the redox mediator is capable of electron exchange with the electrode system. The electrode system, redox mediator and microorganism are understood as discussed elsewhere in this application.

In particular embodiments, the electrochemical device is a disposable, single use device comprising a support, an electrode system as envisaged herein; a redox mediator as envisaged herein, an exogenous microorganism as envisaged herein, and a receptacle configured to contact the fluid sample with the electrode system, the redox mediator and the microorganism, wherein the microorganism is in contact with the redox mediator, wherein the redox mediator is capable of electron exchange with the electrode system, and wherein the electrode system and the receptacle are disposed on the support. In certain embodiments, the receptacle is in the form of a recess or hollow part in the support and/or may be in the form of a porous layer on the support.

The disposable electrochemical device as envisaged herein is particularly adapted to be reversibly inserted in a connection terminal of an electrochemical reader as envisaged herein.

The disposable electrochemical device can be in the form of a single use, disposable electrochemical test strip having a substantially rectangular shape. It shall be appreciated that the electrochemical device can include different forms such as, for example, test strips of different configurations, dimensions or shapes, non-strip test elements, disposable test elements, reusable test elements, micro-arrays, lab-on-chip devices, or other test elements.

As discussed above, in particular embodiments the electrode system comprises one or more working electrodes and a counter electrode and/or a reference electrode. In certain embodiments, the electrode system comprises (i) one or more working electrodes and (ii) a counter electrode, a reference electrode or a counter electrode and a reference electrode. The electrodes are typically manufactured from well-known electrically conductive materials. Carbon- or silver-based electrodes are particularly preferred, in view of their selectivity, ease of manufacture and cost. In certain embodiments, the microorganism and/or the redox mediator may be immobilized on the electrode system. In particular embodiments, the receptacle comprises a test composition as envisaged herein, comprising a redox mediator, a micro-organism and one or more nutrients for the microorganism. In particular embodiments, the receptacle comprises the test composition as envisaged herein in liquid form, in solid form or in gel form, optionally provided with a suitable carrier material, such as cotton, metal particles, paper, polymers or biopolymer, silicates, sponges and the like. In certain embodiments, the receptacle comprises the test composition in solid form, optionally with a suitable carrier material, wherein, upon application of the fluid sample, the test composition rehydrates and dissolves in the liquid sample.

In particular embodiments, the electrode system comprises connecting elements, configured for electrically connecting the electrochemical device as envisaged herein to an electrochemical reader as envisaged herein. In particular embodiments of the electrochemical device, particularly the disposable single-use electrochemical device as envisaged herein, the electrodes and the accompanying connecting elements are mounted on an insulated support. Suitable insulative materials with desirable electrical and structural properties include polycarbonate, glass, ceramic, vinyl polymers, polyimides, polyesters, and the like. The electrodes and accompanying connecting elements may be provided on the support by suitable methods, including but not limited to screen printing methods, ink jet methods, evaporation coating methods or thin film techniques. For instance, commercial graphite printing pastes or commercial silver conducting pastes can be used to produce the electrodes and accompanying connecting elements.

In particular embodiments, the electrochemical device, particularly the disposable single-use electrochemical device, comprises a covering element, adapted for covering the receptacle to avoid evaporation and/or contamination of the receptacle. The cover may be constructed from a wide variety of insulative materials, including but not limited to polycarbonate, glass, ceramics, vinyl polymers, polyimides, polyesters, and the like. In particular embodiments, the electrochemical device, such as the covering element, is provided with an inlet for providing a fluid sample to the electrochemical device.

Figure 2 shows a schematical representation of a non-limiting embodiment of an electrochemical device as envisaged herein, in particular a disposable, single use electrochemical device, configured for insertion in an electrochemical reader, wherein the electrochemical reader is adapted for measuring an electrical signal via the electrode system of the electrochemical device.

The electrochemical device (10) comprises an insulated support (13), whereupon or wherein an electrode system (12), as described herein, and the accompanying connecting elements (11) are deposited. The connecting elements are configured for connecting the electrode system with an electrochemical reader during the analysis of a sample and/or for allowing the electrochemical measurements via the electrode system, such as via a connection terminal of the electrochemical reader. A test composition (14) comprising the redox mediator, the exogenous microorganism and nutrients is situated on the support (13), such as, for instance, in a cavity or in a recessed part within the support, and in contact with the electrode system (12) (FIG 2A and FIG 2B). The sensor (10) further comprises an insulated cover (15), situated on top of the electrochemical device or test composition (FIG 2B), and extending over the test composition (14), thereby avoiding evaporation and/or contamination of the test composition. The cover (15) comprises an inlet (16) for applying the sample to the test composition (14).

The present invention further provides an electrochemical detection system comprising the electrochemical device as envisaged herein, particularly a disposable electrochemical device as envisaged herein, and an electrochemical reader, configured to be in electrical connection and communication with the electrochemical device, particularly via the electrode system and/or the connecting elements. In particular embodiments, the electrochemical reader comprises one or more connection terminals, wherein the disposable electrochemical device as envisaged herein may be inserted in a reversible manner. Multiple connection terminals allow to conduct multiple measurements simultaneously.

In particular embodiments, the electrochemical reader as envisaged herein comprises:
- a power source, such as a battery, configured to be in electrical connection with the electrode system of the electrochemical device and capable of supplying an electrical potential difference between the electrodes sufficient to cause the electro-oxidation of the reduced form of the mediator at the surface of the working electrode; and
- a measuring device in electrical connection with the electrode system and capable of measuring an electrical signal, particularly a current, produced by oxidation of the reduced form of the mediator when an electrical potential difference is applied.

In certain embodiments, the electrochemical detection system further comprises a heating element or thermostatic device, with the aid of which samples can be kept at a pre-set temperature, such as the temperature at which the test organism shows sufficient growth.

In particular embodiments, the electrochemical reader comprises a heating element, particularly situated at the connection terminal, for keeping the biosensor and the sample at a pre-set temperature, particularly at the desired incubation temperature of the microorganism. The electrochemical reader may further comprise a display, particularly an electronic display, for displaying various types of information to the user, such as the measured electrical signal, and a user interface for receiving user input.

In certain embodiments, the electrochemical detection system further comprises a data carrier loaded with a computer program suitable for instructing a computer to analyze digital data obtained from the electrochemical reader. Advantageously, said data carrier loaded with a computer program provides for easy access to the latest available computer programs suitable for use in the method of the present invention.

In certain embodiments, the electrochemical detection system as envisaged herein may further comprise a sampling device, for withdrawing a sample from the fluid to be tested and for adding the sample to the sensor, thereby contacting the sample with the test composition as envisaged herein. A typical sampling device is a container, optionally with volume markings. Examples of a sampling device include a syringe, a pipette or an automated pipetting system.

The present invention further provides for a method of electrochemically detecting an antimicrobial compound in a fluid sample, wherein the sample is introduced in an electrochemical device as envisaged herein, particularly a disposable electrochemical device as envisaged herein.

The methods, electrochemical devices and related systems according to the different embodiments of the present invention are easy to use and implement, in many technical fields. For instance, they can be used in the whole milk production and processing chain, e.g. for self-control in dairy farms, at control points in the milk process industry, or during inspection by authorities. Other food sectors which are vulnerable for misuse of antibacterial agents also benefit from the improved method and means, such as the sea food farming sector producing sea foods like shrimps, salmon, etc.

### Examples

### Example 1. Electrochemical detection of an antimicrobial compound using an oxygen-insensitive, artificial redox mediator

A test composition comprising 0.5 mM Brilliant Black as the redox mediator, LB medium and 1 g/L glucose was introduced in an electrochemical cell of a set up comprising the elements of an electrochemical biosensor as detailed below. The control (A) contained no antibiotic, while the test sample comprised 10 ppm amoxicillin. The analysis was started by introducing 10⁸ *E. coli* CFU/mL to the test compositions with or without the antibiotic compound. The test composition comprising the microorganism was incubated at a temperature of 37°C. A voltage of 0.1 V vs. Ag/AgCl (3 M KCI) was applied to the electrode system.

The measurements were conducted in a thermostated glass cell (purchased from BVT technologies, model TC5) with a carbon screen-printed electrode (ItalSens) used as the working electrode, Pt rod electrode (purchased from BVT technologies, model ACEc) as the counter electrode, and Ag/AgCI (3 M KCI purchased from BVT technologies, model RCEc.R) as the reference electrode. The total volume of the test composition was 10 mL stirred with a magnetic stirrer at 200 rpm.

The results are presented in FIG 3. In the absence of the antibiotic, the current continually increased with increasing incubation time, indicating the uninhibited growth of the microorganism. In contrast, when an antibiotic compound is present in the test composition, the measured current quickly decreased and remained at a low level, demonstrating the inhibiting effect of the antibiotic on the metabolic activity and growth of the microorganism. The difference in the measured current between the control and the sample with the antibiotic compound could already be detected after 30 min of incubation. In contrast, colorimetric detection methods, such as the Delvo^{®} test, requires at least 3 h of incubation time. The difference in current between control and sample quickly increased upon further incubation.

### Example 2. Electrochemical detection of an antimicrobial compound using an oxygen-sensitive, artificial redox mediator

A test composition comprising 0.5 mM 2-hydroxy-1,4-naphthoquinone as redox mediator, LB medium and 1 g/L glucose was introduced in an electrochemical cell as detailed below. The control (A) contained no antibiotic, while the test sample comprised 10 ppm amoxicillin. The analysis was started by introducing 10⁸ *E. coli* CFU/mL to the test compositions with or without the antibiotic compound. The test composition comprising the microorganism was incubated at a temperature of 37°C. A voltage of 0.15 V vs. Ag/AgCI (3 M KCI) was applied to the electrode system.

Measurements were conducted in the thermostated glass cell (purchased from BVT technologies, model TC5) with a carbon screen-printed electrode (ItalSens) used as the working electrode, Pt rod electrode (purchased from BVT technologies, model ACEc) as the counter electrode, and Ag/AgCI (3 M KCI purchased from BVT technologies, model RCEc.R) as the reference electrode. The total volume of the test composition was 10 mL stirred with a magnetic stirrer at 200 rpm.

The results are presented in FIG 4. In the absence of the antibiotic, the measured current initially quickly increased with increasing incubation time, indicating the uninhibited growth of the microorganism. The measured current peaked after about 2 h of incubation, which is presumably due to nutrient exhaustion of the test composition. In the conditions of this example the decay of the current after 2 h can be explained by action of oxygen on the reduced form of the mediator, literally oxidation of the reduced form of the mediator by oxygen which decreases the amount of the mediator that is oxidized at the electrode. When an antibiotic compound is present in the test composition, comparatively small current was measured. Similarly to the measurements in the absence of antibiotics, the current peaks at about 45 min and then drops expectedly due to action of oxygen when bacteria growth and metabolic activity was supressed by the antibiotic. As the growth and metabolic activity of the microorganism was inhibited by the antibiotic, less redox mediator in the reduced form is obtained and more of this form is oxidized by oxygen.

As in example 1, the difference in the measured current between the control and the sample with the antibiotic compound could already be detected after 30 min of incubation and the difference in current between control and sample quickly increased upon further incubation.

### Example 3. Electrochemical detection of an antimicrobial compound using an oxygen-sensitive, artificial redox mediator using a disposable screen-printed electrode system.

Suspension containing 2 × 10⁸ cells of *Lactobacillus rhamnosus* were deposited on a carbon screen-printed electrode (ItalSens) and dried in fridge at 4°C for 4 h. Then, the electrode was inserted in a metallic housing, 0.1 ml of MRS growth medium containing 0.5 mM 2-hydroxy-1,4-naphthoquinone was placed on the screen-printed electrode and covered by a plastic cover. Then a voltage of 0.1 V vs Ag quasi-reference electrode was applied and the housing was warmed until pre-set temperature 37°C. The control (A) contained no antibiotic, while the test sample (B) comprised 10 ppm amoxicillin. The analysis was started by adding the growth medium.

The results are presented in FIG 5. In the presence of the antibiotic, the measured current continuously decreased overtime and dropped more than twice after 1 h of incubation. In the absence of the antibiotic, the measured current stabilized by 1h and started to increase afterwards revealing significant difference in slope of curves in the present and absent of antibiotics. Increase of the current in the absence of the antibiotic after 1 h incubation time corresponds to the observations in example 2 in the absence of the antibiotic. Further optimization of initial amount of bacteria and the deposition procedure is needed to ensure optimal performances of the method.

### Example 4. Electrochemical detection of an antimicrobial compound using potentiometric technique.

A test composition comprising 0.5 mM 2-hydroxy-1,4-naphthoquinone as redox mediator, LB medium and 1 g/L glucose was introduced in an electrochemical cell as detailed below. The control (A) contained no antibiotic, while the test sample comprised 10 ppm amoxicillin. The analysis was started by introducing 3·10⁸ *Lactobacillus rhamnosus* CFU/mL to the test compositions with or without the antibiotic compound. The test composition comprising the microorganism was incubated at a temperature of 37°C.

Measurements were conducted in the thermostated glass cell (purchased from BVT technologies, model TC5) with a carbon screen-printed electrode (ItalSens) used as the working electrode and Ag/AgCI (3 M KCI purchased from BVT technologies, model RCEc.R) as the reference electrode. The open circuit potential between the working electrode and the reference electrode was registered over time. The total volume of the test composition was 10 mL stirred with a magnetic stirrer at 200 rpm.

The results are presented in FIG 6. In the absence of the antibiotic, the measured potential started to decrease from the start of the measurements, whereas the potential remained near the same for first 45 min in the presence of antibiotic. Within 1 h time, a significant difference in the potential value and the slop of the potential as function of time was observed. The decrease in the potential of the working electrode is associated with formation and accumulation of the redox mediator in reduced form at the electrode surface and drop of oxygen concentration in the medium due to bacteria metabolism.

### Example 5. Electrochemical detection of an antimicrobial compound using an oxygen-sensitive, artificial redox mediator - influence of oxygen.

The effect of oxygen when using an oxygen-sensitive redox mediator discussed in examples 2 and 3 above, was further investigated, in particular the possibility for introducing the effect of oxygen in a miniaturized measuring cell without any mechanical stirring of the solution was explored. Chronoamperometric measurements were conducted using disposable screen printed electrodes inserted in a thermostated housing at 37°C according to an embodiment of the invention. The measurements were conducted in the presence of 5 x 10⁸ cells/ml of *Lactobacillus rhamnosus,* 0.5 mM of an oxygen sensitive redox mediator 0.5 mM 2-hydroxy-1,4-naphthoquinone and are represented in FIG 7. Five different volumes of liquid medium were placed in the measurement cell. The volumes were 0.15 ml (curve i), 0.25 ml (curve ii), 0.35 ml (curve iii), 0.5 ml (curve iv), 1 ml (curve v) resulting in 2, 3, 4.5, 6 and 12 mm of calculated liquid levels above the working electrode, respectively. The numbers in the inset denote the levels of the liquid used. Decreasing the liquid level resulted in an increasing influence of air oxygen on the measurements, which was particularly visible at volumes below 0.35 ml (lower than 4.5 mm level of the liquid medium).

### Example 6. Electrochemical detection of an antimicrobial compound in raw milk using an oxygen-sensitive, artificial redox mediator using a disposable screen-printed electrode system.

In another series of experiments, the advantages of the above discussed oxygen effect for detection of an antibiotic compound in undiluted raw cow milk was studied. Chronoamperometric measurements were conducted using disposable screen printed electrodes inserted in a thermostated housing at 37°C according to an embodiment of the invention.

More in particular, a carbon screen-printed electrode (ItalSens) was inserted in a thermostated metallic housing. Then, either 1 ml or 0.35 ml spiked and non-spiked raw cow milk samples was provided on the electrodes. A standard amount of MRS growth medium powder formulation (55 mg/ml - to support the growth of the test organism) and oxygen sensitive mediator 0.5 mM 2-hydroxy-1,4-naphthoquinone were pre-added in the milk. A voltage of 0.1 V vs Ag quasi-reference electrode was applied and the housing was warmed until pre-set temperature 37°C. The control (i) contained no antibiotic (blank milk), while the test sample (ii) comprised 10 ppm oxytetracycline (spiked milk). The analysis was started by adding either 5 x 10⁸ cells/ml or 2.5 x 10⁸ cells/ml of the test organism *Lactobacillus rhamnosus.*

As shown in FIG. 8, in the presence of the antibiotic, the rise of the current impedes compared to the blank sample. When 1 ml of the sample was used, the oxygen mass transfer from air to the near electrode area is hindered, as already shown in FIG 7. Under these conditions, the current in the presence of antibiotic only little differs from the current for the blank control (the current is 30-40% lower compared to blank after 1 h of measurements, FIG 8A). When 0.35 ml of the sample used, the oxygen mass transfer from air to the near electrode area becomes significant as already demonstrated in FIG 7. Under these conditions, the current in the presence of antibiotic substantially differs from the current for the blank control (the current is 40% lower after 20 min and 90% lower after 1 h of measurements, FIG 8B). Under these conditions the difference between the blank control and spiked milk is stronger, which demonstrate the usefulness of the oxygen effect to increase the sensitivity of the method.

The sensitivity can be further enhanced by decreasing starting number of cells of the test microorganisms, as shown in FIG 9. Indeed, a 2-fold decrease in the amount of cells of the test organism results in complete suppression of the current for the milk spiked with the antibiotic. However, decreasing the starting number of cells may increase the analysis time due to slow conversion of the mediator and oxygen at low number of cells. Thus, the starting number of cells and the volume of the liquid sample (level of the liquid above the electrode) should be adjusted to reach a compromise between the required sensitivity and desired analysis time. The analysis time and the sensitivity will be also affected by the nature of the test microorganism.

### Example 7. Redox mediator based electrochemical detection of living microorganisms

A sample of food (0.5 ml raw cow milk obtained from a local farm, normally containing about 10⁴ CFU/ml of bacterial germs according to reports of Milk control centrum of Flanders MCC) or a control sample (0.5 ml sterilized raw cow milk) was mixed with one of three redox mediators, i.e. 0.25 mM 2-hydroxy-1,4-naphthoquinone, 0.25 mM 2-methyl-1,4-naphthoquinone or 0.25 mM brilliant black, and incubated at 37°C using the setup with a carbon screen-printed electrode (ItalSens) as in examples 3, 5 and 6. A voltage of 0.1 V vs. Ag reference electrode was applied to the electrode system in the case of 2-hydroxy-1,4-naphthoquinone and 2-methyl-1,4-naphthoquinone or 0.2 V vs. Ag reference electrode in the case of brilliant black. The analysis was started by placing the samples and controls in the measuring setup. The current was registered as function of time (FIG 10). Additional nutrients (such as glucose, LB, MRS or another growth medium formulations) can be added in the solution to stimulate a microorganism growth and thus possibly detect its growth in a shorter time.

Alternatively, samples and controls can be analysed in the electrochemical setup described in examples 1, 2 and 4. Such setup consists of a thermostated glass cell (purchased from BVT technologies, model TC5) with a carbon screen-printed electrode (ItalSens) used as the working electrode, Pt rod electrode (purchased from BVT technologies, model ACEc) as the counter electrode, and Ag/AgCI (3 M KCI purchased from BVT technologies, model RCEc.R) as the reference electrode. The total volume of the test composition is 10 mL stirred with a magnetic stirrer at 200 rpm. The analysis of samples starts by adding the samples into the test composition such as LB medium supplemented with 1 g/L glucose or MRS. The test composition also contains a redox mediator, such as 0.25 mM 2-hydroxy-1,4-naphthoquinone or 0.25 mM brilliant black. Then the mixture of the test composition and samples is incubated at a temperature of 37°C and the electrochemical response is measured as function of time. As shown in FIG. 10, the growth in the sample is detected after 12 h in the case of 2-hydroxy-1,4-naphthoquinone and 2-methyl-1,4-naphthoquinone and after 20 h for brilliant black. The growth is detected as the rise of current in time. No rise of the current is seen in the sterile controls. The results clearly indicates that the suggested setup can detect growth of bacteria present in a food sample.

## Claims

1. A method of electrochemically detecting microbial growth comprising
(a) contacting a fluid sample with an electrode system and a redox mediator, wherein the sample is in contact with the redox mediator and wherein the redox mediator is capable of electron exchange with the electrode system;
(b) incubating the sample under conditions suitable for the growth of a microorganism; and
(c) measuring an electrical signal, preferably a potential or current output, via the electrode system, wherein the measured electrical signal is a function of the growth of a microorganism in said fluid sample.

2. The method of claim 1 which is a method of electrochemically detecting an antimicrobial compound in a fluid sample comprising
(a) contacting the fluid sample with an electrode system, a redox mediator, and an exogenous microorganism, wherein the fluid sample and the exogenous microorganism are in contact with the redox mediator and wherein the redox mediator is capable of electron exchange with the electrode system;
(b) incubating the sample under conditions suitable for the growth of the exogenous microorganism;
(c) measuring an electrical signal via the electrode system, wherein the measured electrical signal is a function of the growth of the exogenous microorganism; and
(d) correlating the measured electrical signal with the presence or absence of an antimicrobial compound in the sample.

3. The method according to claim 1 or 2, wherein step (c) comprises the step of
(i) applying a voltage to the electrode system and measuring the current output, wherein the measured current output is a function of the growth of a microorganism when present in the fluid sample or of the inhibition thereof when an antimicrobial compound is present in the fluid sample ; or
(ii) measuring the electrical potential between the electrodes, wherein the measured potential is a function of the growth of a microorganism when present in the fluid sample or of the inhibition thereof when an antimicrobial compound is present in the fluid sample.

4. The method according to any one of claims 1 to 3, wherein step (c) and step (b) are performed simultaneously, particularly for a period between 15 min and 3 hr, more particularly for a period between 30 min and 120 min.

5. The method according to any one of claims 1 to 4, wherein the redox mediator is an oxygen-sensitive redox mediator, preferably 2-hydroxy-1,4-naphthoquinone or a naphthoquinone derivative, phenazine methosulfate or a phenazine derivative.

6. The method according to any one of claims 1 to 4, wherein the redox mediator is an oxygen-insensitive redox mediator, preferably brilliant black, benzoquinone or a benzoquinone derivative, naphthoquinone or 2-methyl-1,4,-naphthoquinone, resazurin or a phenoxazine derivative, thionine or a phenothiazine derivatives, isoalloxazine or a flavin derivative, ferrocene or a ferrocene derivative, Osmium bis(2,2'-bipyridine)chloride or an Osmium-containing complex including a polymeric substance.

7. The method of electrochemically detecting an antimicrobial compound in a fluid sample according to any one of claims 2 to 6 wherein the exogenous microorganism is a mesophilic non-spore forming microorganism, such as *Escherichia* sp. or a fermenting lactic acid bacteria, or wherein the microorganism is a thermophilic microorganism, such as a thermophilic *Bacillus* sp. or *Geobacillus* sp.

8. The method of electrochemically detecting an antimicrobial compound in a fluid sample according to any one of claims 2 to 7, wherein the exogenous microorganism and/or the redox mediator is immobilized on the electrode system.

9. The method according to any one of claims 1 to 8 wherein in step (a) the fluid sample is contacted with one or more nutrients suitable for the growth of a microorganism.

10. The method according to any one of claims 1 to 91, wherein the fluid sample is a fluid sample originating from a body liquid, an animal product, an animal tissue, a food product derived from an animal product or an animal tissue, or an aqueous environmental liquid, preferably the sample is blood, urine, wastewater or milk, more preferably milk.

11. An electrochemical device for detecting an antimicrobial compound in a fluid sample, comprising an electrode system, a redox mediator, a microorganism, and a receptacle configured to contact the fluid sample with the electrode system, the redox mediator and the microorganism; wherein the microorganism is in contact with the redox mediator and wherein the redox mediator is capable of electron exchange with the electrode system.

12. An electrochemical device according to claim 11, wherein the electrochemical device is a disposable device, further comprising a support, wherein the electrode system and the receptacle is disposed on the support.

13. An electrochemical device according to claim 12, wherein the receptacle comprises a composition comprising a redox mediator, preferably an oxygen sensitive redox mediator, a microorganism and one or more nutrients for said microorganism.

14. An electrochemical detection system comprising the electrochemical device according to any one of claims 11 to 13 and an electrochemical reader, wherein the electrochemical reader is configured to interact with the electrochemical device according to any one of claims 11 to 13, particularly wherein the electrochemical reader is adapted for the introduction of the electrochemical device according to any one of claims 11 to 13 within the electrochemical reader.

15. The electrochemical detection system of claim 14, further comprising:
- a heating element for adjusting the incubation temperature of the electrochemical device according to any one of claims 11 to 13, particularly wherein the electrochemical reader comprises a heating element, adapted for heating the electrochemical device when the electrochemical device is introduced in the electrochemical reader; and/or
- a sampling device for withdrawing a sample from a fluid to be tested and for adding the fluid sample to the electrochemical device according to any one of claims 11 to 13.
